# EUROPEAN PATENT SPECIFICATION

(11) **EP 3 366 153 B1**
(45) Date of publication and mention of the grant of the patent: **07.08.2019**
(21) Application number: 18154344.8
(22) Date of filing: 31.01.2018
(51) Int. Cl.: A42B 3/18, A61F 9/02, G02C 3/02

(54) **PROTECTIVE HELMET WITH FLIP-UP VISOR/GOGGLES**
SCHUTZHELM MIT HOCHKLAPPBAREM VISIER/BRILLE
CASQUE DE PROTECTION À LUNETTES/VISIÈRE RABATTABLES

(30) Priority: 24.02.2017 IT 201700021390
(43) Date of publication of application: 29.08.2018
(73) Proprietor: Ci.Erre.E. S.R.L., 24030 Brembate di Sopra (BG) (IT)
(72) Inventor: Pilenga, Paolo, I-24040 Stezzano (BG) (IT)
(74) Representative: Giavarini, Francesco

(56) References cited:
- CA-A1- 2 767 266
- US-A1- 2014 173 811

## Description

The present invention relates to a protective helmet provided with a mask/goggles movable between a position of use and one of non-use. More in particular, the present invention relates to a protective helmet provided with a mask/goggles integrated with the shell of said helmet so as to be easily movable between a position of use and one of non-use in which it nonetheless remains fixed to said shell.

In certain jobs or in certain sports, such as cycling, skiing or climbing, it is known to use protective helmets to guarantee safety during performance of the job or sport.

In frequent cases, the use of a protective helmet can be accompanied by the use of a mask or goggles that can have the function of protecting the eyes from foreign bodies (e.g. from splinters in the case of particular jobs, insects for example in the case of cycling, and similar problems) and/or of protecting the eyes from the sun/light. In this case, the combined use of the helmet and goggles can pose various problems.

For example, the use of normal eyewear with the arms inserted in the space between skull and shell of the helmet is usually annoying given that, in the long term, the pressure exerted by the arm of the eyewear pressed against the head of the user becomes painful.

Another possibility, typically used for example in the skiing field is that of using a mask provided with an elastic strap that is worn over the helmet so as to adhere to the outer wall of the shell with the mask positioned at the level of the eyes and held in position by the tensile force exerted by the elastic strap.

To prevent the mask from being accidentally removed, for example in the case of impact, the elastic strap can be blocked by appropriate means provided on the shell, usually consisting of a slot with a flap fixed removably to the helmet, which is detached from the shell to allow positioning of the elastic strap and then reattached to hold it in place. In case of impact, or in case of removal of the elastic strap from the shell of the helmet, the mask remains hanging in the slot in which it is inserted.

However, this solution has various disadvantages. For example, the tension exerted by the elastic strap maintains the mask pressed against the face of the user, which can become bothersome in the long term. Moreover, although masks can be provided with openings appropriate to ensure the passage of air in particular weather/environmental conditions, their use can become uncomfortable. A further drawback is given by the fact that the mask can shift following an impact or other accidental events, creating discomfort for the user or even limiting or obstructing the field of vision. Finally, for correct positioning of the mask it is normally necessary to use both hands and this can be difficult, if not impossible, in specific conditions or moments.

In the case in which environmental conditions vary continuously and rapidly, or in the case in which it is necessary to put on and remove the mask frequently (for example when it is used as protective mask for specific jobs), management of the mask or goggles combined with the helmet can become complicated and cumbersome and at least one free hand is required to hold the mask/goggles.

Various solutions have been proposed over time, such as the production of helmets in which the mask is integrated retractably in a gap produced inside the shell.

From a technical point of view, this solution has a certain complexity, as the space inside the helmet must be sized appropriately also to house the mask and produce the related operating mechanism. Moreover, this solution is difficult to implement in light helmets, such as cycling helmets, as the structure of the shell cannot be weakened by the production of spaces and cavities inside it, also taking account of the fact that to accommodate a mask, however small it is, the related seat will have a width substantially the same as the width of the helmet and a depth the same as the height of the mask. Moreover, document US 2014/0173811 A1 describes a helmet, corresponding to the preamble of claim 1, and including a mask held in place by magnetic devices. This solution however, does not solve all the problems already mentioned.

Therefore, it would be desirable to have a system comprising a protective helmet and a mask/goggles, which is capable of overcoming the problems related to known systems. Therefore, an object of the present invention is to provide a protective helmet that can easily integrate the use of a mask/goggles.

A further object of the present invention is to provide a protective helmet that allows easy positioning in use of a mask/goggles, even using only one hand.

Another object of the present invention is that of providing a system comprising a protective helmet and a mask/goggles in which the possibility of losing the mask/goggles is prevented or limited.

Yet another object of the present invention is to provide a system comprising a protective helmet and a mask/goggles that allows easy and rapid positioning of the mask/goggles in the operating position.

A further object of the present invention is to provide a system comprising a protective helmet and a mask/goggles in which it is possible to put on and remove the mask/goggles easily, even very frequently.

One more object of the present invention is to provide a system comprising a protective helmet and a mask/goggles that is easy to produce at competitive costs.

The aforesaid and other objects and advantages and others, which will be apparent from the description below, are achieved by means of a protective helmet as defined in the independent claim 1, in particular for jobs or sporting activities such as cycling and skiing, comprising a shell adapted to surround a portion of the skull of a user, a mask/goggles adapted to protect at least a portion of the face at the eyes of a user and connection means between said shell and said mask/goggles, characterized in that said connection means comprise a first and a second rigid element constrained respectively, at their first end, to a first and a second side portion of said mask/goggles and pivoted respectively, at their second end, on a first and a second side portion of said shell, said connection means being adapted to implement a movement in translation and rotation of said mask/goggles with respect to said shell between a first position in which said mask/goggles is/are positioned at a lower front edge of said shell and a second position in which said mask/goggles is/are positioned adjacent to a rear portion of said shell.

In this way, a protective helmet that satisfies the aforesaid objects is obtained.

In particular, the mask/goggles is/are always fixed to the helmet safely without the risk of accidental movement or falling.

Moreover, the helmet according to the present invention does not require devices, such as the arms of the goggles, interposed between helmet and head of the user and which can cause the user discomfort. The fixing and movement system of the mask/goggles is in fact outside the shell and therefore does not cause any problems for the user.

The movement of the visor between the position of use and non-use is very simple and can be carried out rapidly even with one hand.

Positioning of the connection means between the shell and the mask/goggles does not require particular modifications to the structure of the shell.

In the position of use the mask/goggles is/are not subject to unwanted movements even following impacts or accidental events, being maintained in position by the rigidity of the connection means, besides the fact that as the visor is accommodated under the front edge of the helmet it cannot move upward.

Advantageously, said connection means comprise first and second spring means that connect said first and second side portion of said shell respectively to said first and second rigid element. As better explained below, the spring means allow the mask/goggles to be maintained in position in the first or in the second position.

Preferably, said connection means comprise complementary guide means positioned on said first and second side portion of said shell and on said first and second rigid element.

In this case, said complementary guide means comprise a first and a second groove positioned respectively on said first and second rigid element, and a first and a second pin positioned respectively on said first and second side portion of said shell and slidingly inserted respectively in said first and second groove. In practice, according to this embodiment and as better explained below, the first and second pin form the fixed point along which the first and second rigid element can slide and rotate.

Advantageously, said first spring means are connected to a said first pin and to said first rigid element and said second spring means are connected to said second pin and to said second rigid element.

In this embodiment, preferably said first and second rigid element each comprise a fixing pin and a housing seat for the respective first and second spring means.

In order to facilitate the rotation of the first and second rigid element, said first and second pin are positioned respectively on a first and second bushing mounted in free rotation respectively about a third and fourth pin fixed to said shell, said first and second bushing being inserted and free to slide respectively in said first and second groove. In practice, the first and second bushing form the interface between the pins fixed to the shell and the first and second rigid element, facilitating their translation and rotation with respect to the shell.

In a particularly preferred embodiment of a protective helmet according to the present invention said connection means comprise a first and a second guide plate comprising an abutment surface respectively of said first and second rigid element. Said plate and abutment surface have the purpose of maintaining the first and second rigid element (and consequently the mask/goggles) in a fixed position when the mask/goggles is/are in the first and in the second position, and of guiding the translation and rotation movement of the first and second rigid element during passage between the two positions.

In this case, advantageously, said abutment surface protrudes laterally from said plate and is adapted to cooperate with an upper and lower edge of the corresponding first or second rigid element.

In a preferred embodiment of the protective helmet according to the present invention, in order to improve the aerodynamic properties and provide a suitable aesthetic appearance, said shell comprises a housing seat of said mask/goggles positioned in said rear portion of said shell at said second position of said mask/goggles.

Further characteristics and advantages of the present invention will be more apparent from the description of the preferred embodiment, illustrated by way of non-limiting example in the accompanying figures, wherein:

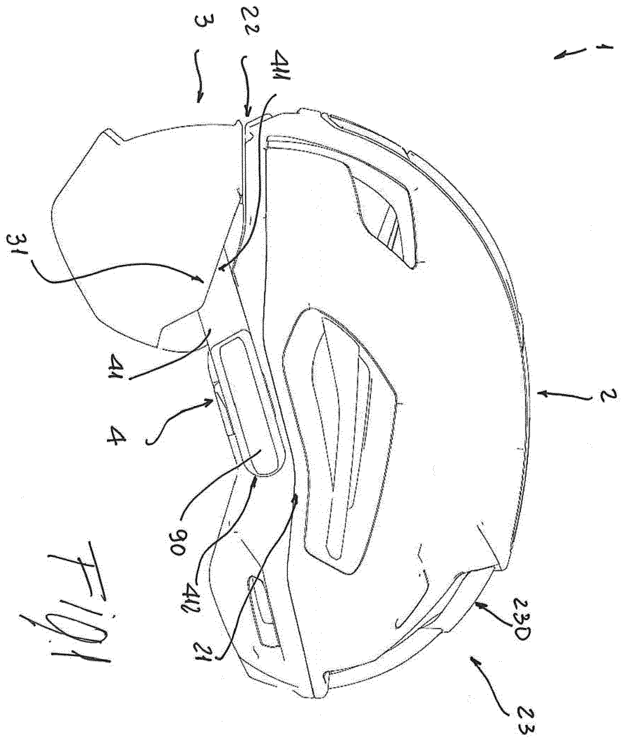

- Fig. 1 is a side view of an embodiment of a helmet according to the present invention, with the mask/goggles in a first position;

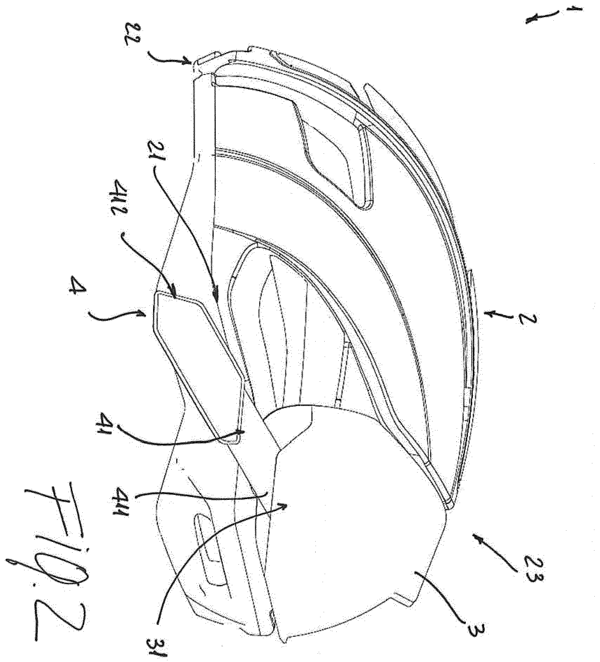

- Fig. 2 is a side view of an embodiment of a helmet according to the present invention, with the mask/goggles in a second position;

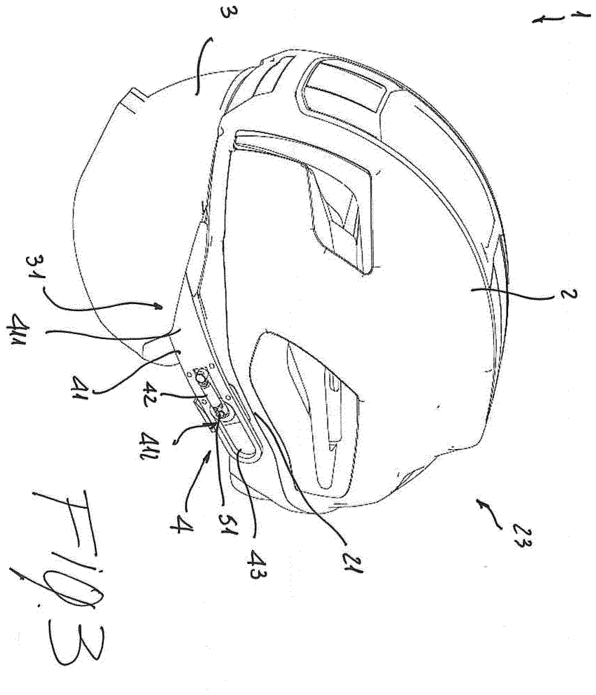

- Fig.3 is a perspective view of an embodiment of a helmet according to the present invention, with the mask/goggles in a first position;

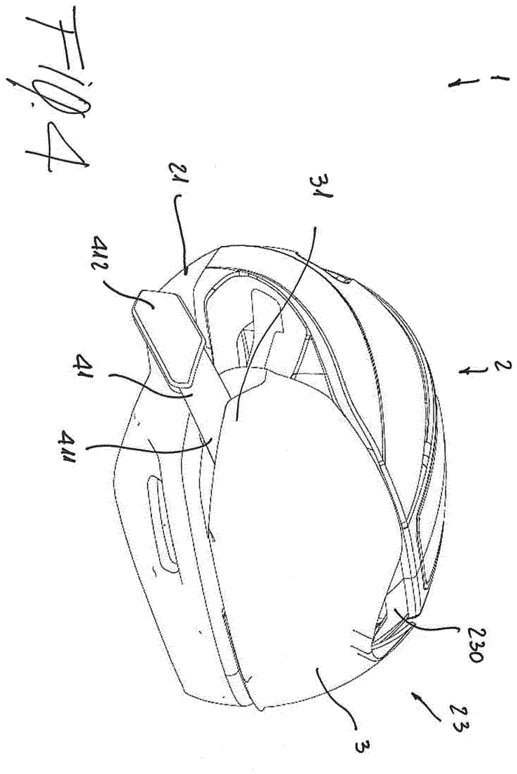

- Fig. 4 is a perspective view of an embodiment of a helmet according to the present invention, with the mask/goggles in a second position;
- Fig. 5 is a side view of the embodiment of Fig. 1 that illustrates some details of the connection means between the shell and the mask/goggles;

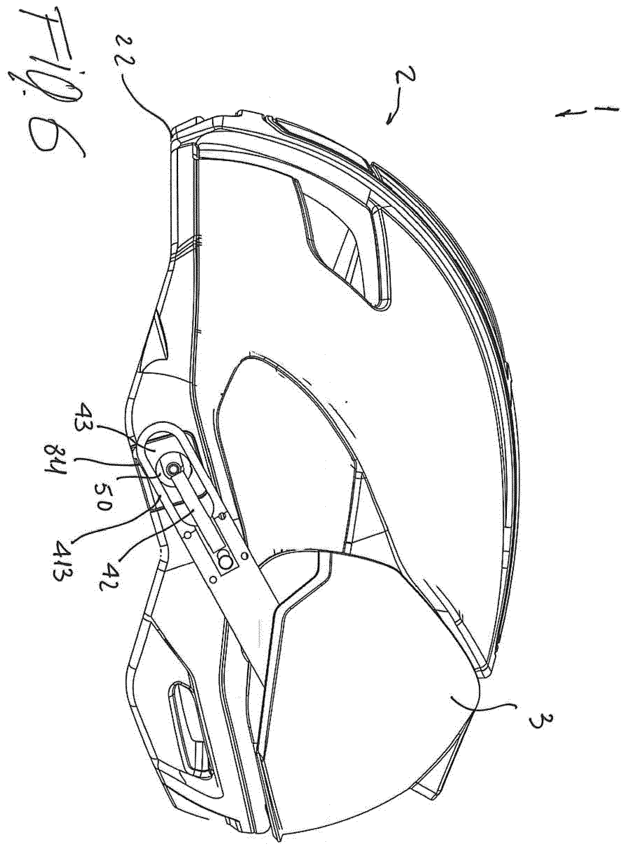

- Fig. 6 is a side view of the embodiment of Fig. 2 that illustrates some details of the connection means between the shell and the mask/goggles 3;
- Fig. 7 is a side view of an embodiment of a helmet according to the present invention that illustrates some details of the connection means between the shell and the mask/goggles in an intermediate position between the positions of Figs. 5 and 6;
- Fig. 8 is a perspective exploded view that illustrates various components of the connection means between the shell and the mask/goggles.

In the accompanying figures, the helmet is visible only from one side and the description is provided with numerical references relating only to the visible side. Naturally, given the structural symmetry it is understood that the elements cited are reproduced and present also on the other side, unless otherwise specified.

With reference to the accompanying figures, a protective helmet according to the present invention, defined with the reference numeral 1, comprises - in its most general embodiment - a shell 2 adapted to surround a portion of the skull of a user, a mask/goggles 3 adapted to protect at least a portion of the face at the eyes of a user and connection means 4 between said shell 2 and said mask/goggles 3.

One of the peculiar characteristics of the protective helmet 1 according to the present invention is given by the fact that said connection means 4 comprise a first 41 and a second rigid element that are constrained respectively, at their first end 411, to a first 31 and a second side portion of said mask/goggles 3.

The first 41 and second rigid element are then pivoted respectively, at their second end 412, on a first 21 and a second side portion of said shell 2.

A further peculiar characteristic of the protective helmet 1 according to the present invention is given by the fact that said connection means 4 are adapted to implement a movement in translation and rotation of said mask/goggles 3 with respect to said shell 2 between a first operating position, of use, in which said mask/goggles 3 is/are positioned at a lower front edge 22 of said shell and a second operating position, of non-use, in which said mask/goggles 3 is/are positioned adjacent to a rear portion 23 of said shell 2.

In an embodiment of a protective helmet 1 according to the present invention said connection means 4 comprise first 42 and second spring means that connect said first 21 and second side portion of said shell 2 respectively to said first 41 and second rigid element.

In practice, the connection between shell 2 and mask/goggles 3 is implemented by rigid coupling elements so as to ensure stability of position to the mask/goggles 3 in the two operating positions. The rigid coupling elements can move in a manner guided in translation and rotation (as better described below), but this movement is not implemented freely as opposed by the spring means that oblige the first 41 and the second rigid element to remain in the first or in the second position. The movement is only possible with the intervention of an external force, for example by a user who moves the mask/goggles 3 overcoming the resistance force of the spring that opposes the motion.

Advantageously, to facilitate movement of the mask/goggles 3, said connection means 4 comprise complementary guide means positioned on said first 21 and second side portion of said shell 2 and on said first 41 and second rigid element. In particular, said complementary guide means comprise a first 43 and a second groove that are positioned respectively on said first 41 and second rigid element, and a first 51 and a second pin that are positioned respectively on the first 21 and second side portion of said shell 2 and that are slidingly inserted respectively in said first 43 and second groove.

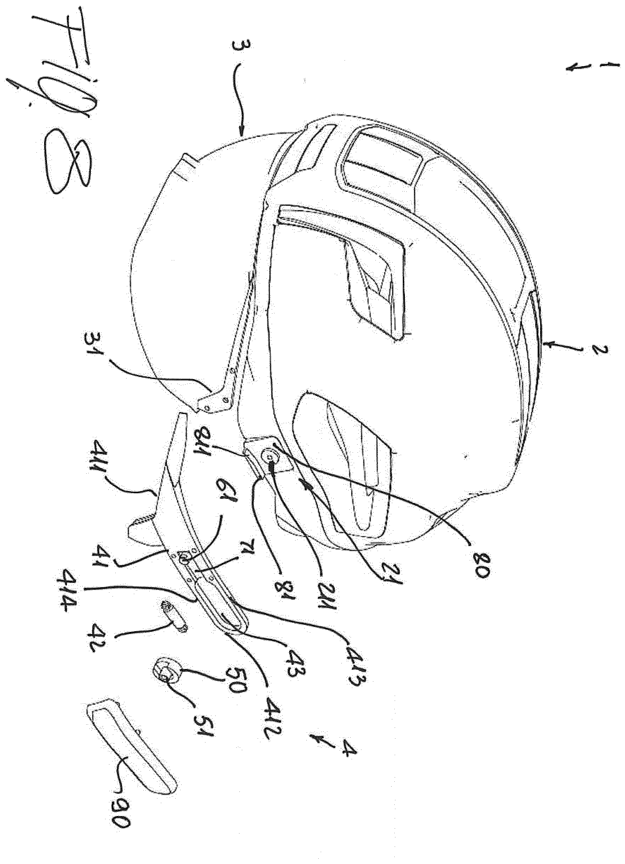

As can be seen in the accompanying figures, in particular in Fig. 8, in the embodiment represented said first spring means 42 are connected to said first pin 51 and to said first rigid element 41 and said second spring means are connected to said second pin and to said second rigid element.

Maintaining in position and a related reduction of the overall dimensions can be obtained if the first 41 and the second rigid element each comprise a fixing pin 61 on which the first 42 and second spring means are fixed, and a housing seat 71, for example formed of a groove or recess in the first 41 and second rigid element, said housing seat 7 being adapted to at least partially accommodate the respective first 42 and second spring means.

In a particular embodiment of a protective helmet 1 according to the present invention, adapted to facilitate the translation and rotation movement of the first 41 and second rigid element, said first 51 and second pin are positioned respectively on a first 50 and second bushing that are mounted in free rotation respectively about a third 211 and fourth pin fixed to said shell 2, the first 50 and second bushing being inserted and free to slide respectively in said first 43 and second groove

According to a particularly preferred embodiment of the protective helmet 1 of the present invention, said connection means comprise a first 80 and a second guide plate, each of which comprises an abutment surface 81 respectively of said first 41 and second rigid element. Preferably, said abutment surface 81 protrudes laterally from said plate 80 and is adapted to cooperate with an upper 413 and lower 414 edge of the corresponding first 41 or second rigid element, according to an operating principle described below.

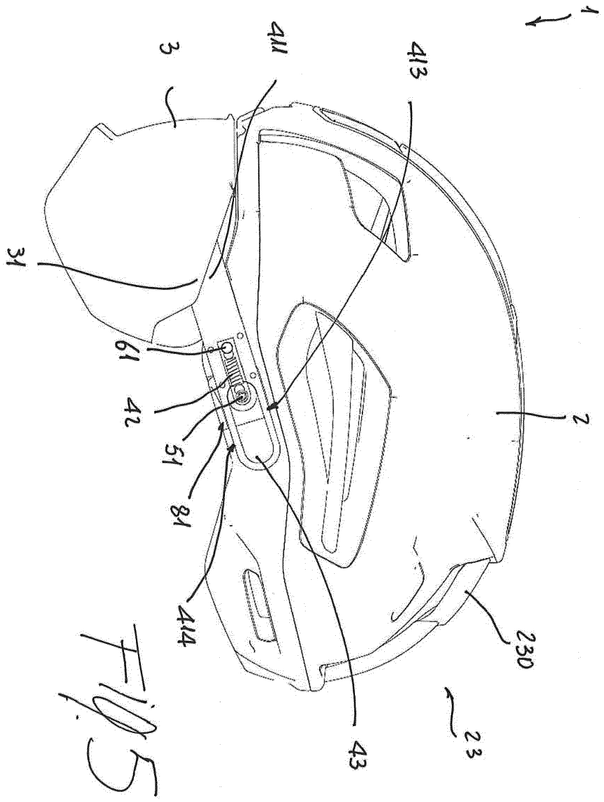

Fig. 5 represents a protective helmet 1 according to the present invention in the first position of use. In this position, the mask/goggles 3 is/are positioned at a lower front edge 22 of said shell so as to shield/protect the eyes of a user.

In this configuration the spring means 42 act on the corresponding rigid element 41 exerting thereon an inward tensile force. In this way, the rigid element 41 (and consequently the mask/goggles 3) is held in substantially fixed position by the abutment offered to the tension of the spring by the pin 51 against the end wall of the groove 43, and by the abutment between the abutment surface 81 of the guide plate obtained against the lower edge 414 of the first rigid element 41 (analogous situation occurs on the opposite side, not illustrated).

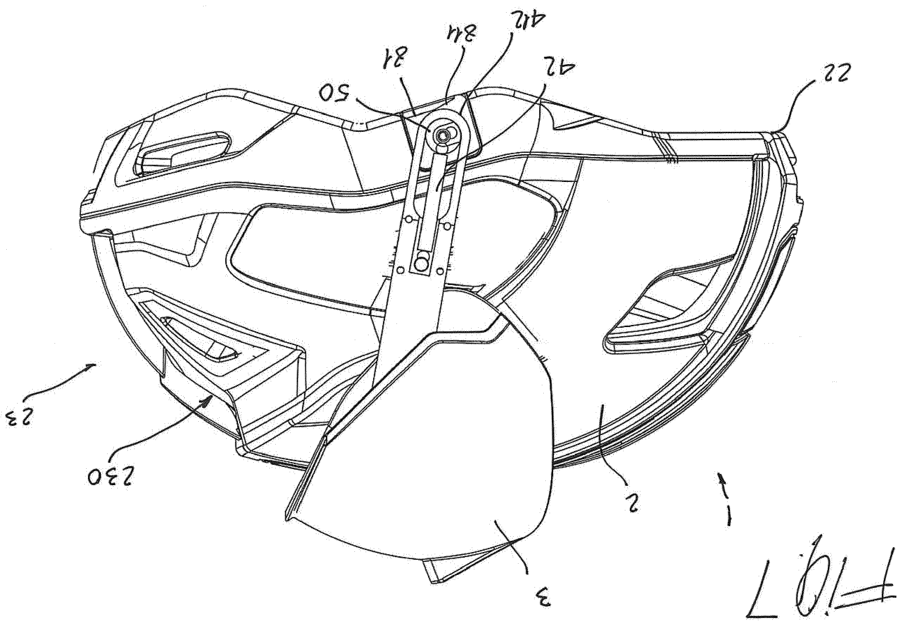

To implement the movement of the mask/goggles 3 between the first and the second position, the user exerts a tension on the mask/goggles 3 causing it/them to move forward. In this first phase the movement is a rectilinear forward movement and is guided by the sliding of the pin 51 in the groove 43 and of the lower edge 414 of the first rigid element 41 on the abutment surface 81. The sliding of the pin 51 in the groove 43 terminates when the pin abuts against the end of the groove 43 proximal to the second end 412 of the first rigid element 41. At this point, sliding of the lower edge 414 of the first rigid element 41 on the abutment surface 81 is also completed and the first rigid element 41 is free to rotate about the pin 51. Rotation takes place maintaining the spring 42 in traction and is partly guided by the sliding of the rounded second end 412 of the first rigid element 41 on the abutment surface 81, which allows the mask/goggles 3 to be maintained at a suitable distance from the shell 2, as shown in Fig. 7. In proximity of the second position of the mask/goggles 3 the rounded second end 412 of the first rigid element 41 is released from the abutment surface 81 and the first rigid element 41 can start to slide toward the front of the helmet as a result of the tension of the spring 42 and causing insertion of the mask/goggles 3 in a housing seat 230 positioned in the rear portion 23 of said shell 2. This movement is guided by sliding of the upper edge 413 of the first rigid element 41 on a suitably inclined plane 811 of the abutment surface 81. This movement terminates when the mask/goggles 3 is/are housed in said housing seat 230 at said second position of said mask/goggles 3.

The mask/goggles 3 is/are maintained in position by the tension force of the spring 42, adhering to the rear portion 23 of the shell 2.

The closing movement of the mask/goggles 3 takes place according to a reverse operating sequence with regard to the one described.

Advantageously, the helmet 1 according to the present invention can also be provided with further accessory elements, such as a closing cover 90 to protect part of the components of the connection means 4.

It is evident how the protective helmet described here allows the objects and advantages set forth above to be obtained. The protective helmet according to the present invention can advantageously applied to various fields of sporting practice and to jobs in which the use of protective means for the head and the eyes of the user is required or advantageous.

On the basis of the description provided, other characteristics, modifications or improvements are possible and evident to a person skilled in the art. These characteristics, modifications and improvements should therefore be considered part of the present invention that is only limited by the appended claims. In practice, the materials used, the dimensions and contingent shapes can be any according to requirements and to the state of the art.

## Claims

1. Protective helmet (1) comprising a shell (2) adapted to surround a portion of the skull of a user, a mask/goggles (3) adapted to protect at least a portion of the face at the eyes of a user and connection means (4) between said shell (2) and said mask/goggles (3), **characterized in that** said connection means (4) comprise a first (41) and a second rigid element constrained respectively, at their first end (411), to a first (31) and a second side portion of said mask/goggles (3) and respectively pivoted, at their second end (412), on a first (21) and a second side portion of said shell (2), said connection means (4) being adapted to implement a movement in translation and rotation of said mask/goggles (3) with respect to said shell (2) between a first position in which said mask/goggles (3) is/are positioned at a lower front edge (22) of said shell (2) and a second position in which said mask/goggles (3) is/are positioned adjacent to a rear portion (23) of said shell (2).

2. Protective helmet (1) according to claim 1, **characterized in that** said connection means (4) comprise first (42) and second spring means which connect said first (21) and second side portion of said shell (21) respectively to said first (41) and second rigid element.

3. Protective helmet (1) according to claim 1 or 2, **characterized in that** said connection means (4) comprise complementary guide means positioned on said first (21) and second side portion of said shell (2) and on said first (41) and second rigid element.

4. Protective helmet (1) according to claim 3, **characterized in that** the said complementary guide means comprise a first (43) and a second groove positioned respectively on said first (41) and second rigid element, and a first (51) and a second pin positioned respectively on said first (21) and second side portion of said shell (2) and slidingly inserted respectively in said first (43) and second groove.

5. Protective helmet (1) according to claims 2 and 4, **characterized in that** said first spring means (42) are connected to said first pin (51) and said first rigid element (41) and said second spring means are connected to said second pin and said second rigid element.

6. Protective helmet (1) according to claim 5, **characterized in that** said first (41) and second rigid element each comprise a fixing pin (61) and a housing seat (71) for the respective first (42) and second spring means.

7. Protective helmet (1) according to claim 6, **characterized in that** said first (51) and second pin are respectively positioned on a first (50) and second bushing mounted in free rotation respectively about a third (211) and fourth pin fixed to said shell (2), said first (50) and second bushing being inserted and free to slide respectively in said first (43) and second groove.

8. Protective helmet (1) according to one or more of the preceding claims, **characterized in that** said connection means comprise a first (80) and a second guide plate comprising an abutment surface (81) respectively of said first (41) and second rigid element.

9. Protective helmet according to claim 8, **characterized in that** said abutment surface (81) protrudes laterally from said plate (80) and is adapted to cooperate with an upper (413) and lower (414) edge of the corresponding first (41) or second rigid element.

10. Protective helmet (1) according to one or more of the preceding claims, **characterized in that** said shell (2) comprises a housing seat (230) of said mask/goggles (3) positioned in said rear portion (23) of said shell (2) at said second position of said mask/goggles (3).

## Patentansprüche

1. Schutzhelm (1), umfassend eine Schale (2), die ausgelegt ist, um einen Teil des Schädels eines Benutzers zu umgeben, eine Maske/Schutzbrille (3), ausgelegt zum mindestens teilweise Schützen eines Teil des Gesichts an den Augen eines Benutzers, und Verbindungsmittel (4) zwischen der Schale (2) und der Maske/Schutzbrille (3), **dadurch gekennzeichnet, dass** die Verbindungsmittel (4) ein erstes (41) und ein zweites starres Element umfassen, die jeweils eingeschränkt sind, an ihrem ersten Ende (411), zu einem ersten (31) und einem zweiten Seitenteil der Maske/Schutzbrille (3) und jeweils geschwenkt sind, an ihrem zweiten Ende (412), an einem ersten (21) und einem zweiten Seitenteil der Schale (2), wobei die Verbindungsmittel (4) ausgelegt sind, um eine Übersetzungs- und Drehbewegung der Maske/Schutzbrille (3) in Bezug auf die Schale (2) zwischen einer ersten Position, in der die Maske/Schutzbrille (3) an einem unteren vorderen Rand (22) der Schale (2) positioniert ist/sind, und einer zweiten Position, in der die Maske/Schutzbrille (3) benachbart eines hinteren Teils (23) der Schale (2) positioniert ist/sind, umzusetzen.

2. Schutzhelm (1) nach Anspruch 1, **dadurch gekennzeichnet, dass** die Verbindungsmittel (4) erste (42) und zweite Federmittel umfassen, die das erste (21) und das zweite Seitenteil der Schale (21) jeweils mit dem ersten (41) und dem zweiten starren Element verbinden.

3. Schutzhelm (1) nach Anspruch 1 oder 2, **dadurch gekennzeichnet, dass** die Verbindungsmittel (4) ergänzende Führungsmittel umfassen, die an dem ersten (21) und zweiten Seitenteil der Schale (2) und an dem ersten (41) und zweiten starren Element positioniert sind.

4. Schutzhelm (1) nach Anspruch 3, **dadurch gekennzeichnet, dass** die komplementären Führungsmittel eine erste (43) und eine zweite Nut umfassen, die jeweils an dem ersten (41) und dem zweiten starren Element positioniert sind, sowie einen ersten (51) und einen zweiten Stift, die jeweils an dem ersten (21) und dem zweiten Seitenteil der Schale (2) positioniert sind und gleitend jeweils in die erste (43) und die zweite Nut eingeführt werden.

5. Schutzhelm (1) nach einem der Ansprüche 2 und 4, **dadurch gekennzeichnet, dass** die ersten Federmittel (42) mit dem ersten Stift (51) und dem ersten starren Element (41) verbunden sind und die zweiten Federmittel mit dem zweiten Stift und dem zweiten starren Element verbunden sind.

6. Schutzhelm (1) nach Anspruch 5, **dadurch gekennzeichnet, dass** das erste (41) und das zweite starre Element jeweils einen Befestigungsstift (61) und einen Gehäusesitz (71) für das erste (42) bzw. zweite Federmittel umfassen.

7. Schutzhelm (1) nach Anspruch 6, **dadurch gekennzeichnet, dass** der erste (51) und der zweite Stift jeweils an einer ersten (50) und einer zweiten Buchse positioniert sind, die jeweils um einen dritten (211) und einen vierten Stift, die an der Schale (2) befestigt sind, frei drehbar angebracht sind, wobei die erste (50) und die zweite Buchse eingeführt sind und frei in der ersten (43) bzw. zweiten Nut gleiten können.

8. Schutzhelm (1) nach einem oder mehreren der vorstehenden Ansprüche, **dadurch gekennzeichnet, dass** die Verbindungsmittel eine erste (80) und eine zweite Führungsplatte umfassen, die eine Anlagefläche (81) des ersten (41) bzw. zweiten starren Elements umfassen.

9. Schutzhelm nach Anspruch 8, **dadurch gekennzeichnet, dass** die Anlagefläche (81) lateral von der Platte (80) vorsteht und ausgelegt ist, um mit einem oberen (413) und einem unteren (414) Rand des entsprechenden ersten (41) oder zweiten starren Elements zusammenzuwirken.

10. Schutzhelm (1) nach einem oder mehreren der vorstehenden Ansprüche, **dadurch gekennzeichnet, dass** die Schale (2) einen Gehäusesitz (230) der Maske/Schutzbrille (3) umfasst, der in dem hinteren Teil (23) der Schale (2) an der zweiten Position der Maske/Schutzbrille (3) positioniert ist.

## Revendications

1. Casque de protection (1) comprenant une coque (2) adaptée pour entourer une partie du crâne d'un utilisateur, un masque/des lunettes de protection (3) adapté(es) pour protéger au moins une partie du visage au niveau des yeux d'un utilisateur et un moyen de raccordement (4) entre ladite coque (2) et le(s)dit(es) masque/lunettes de protection (3), **caractérisé en ce que** ledit moyen de raccordement (4) comprend un premier (41) et un second éléments rigides respectivement contraints, à leur première extrémité (411), à une première (31) et une seconde parties latérales dudit/desdites masque/lunettes de protection (3) et respectivement pivotés, à leur seconde extrémité (412), sur une première (21) et une seconde parties latérales de ladite coque (2), ledit moyen de raccordement (4) étant adapté pour mettre en oeuvre un mouvement en translation et rotation dudit/desdites masque/lunettes de protection (3) par rapport à ladite coque (2) entre une première position dans laquelle le(s)dit(es) masque/lunettes de protection (3) est/sont positionné(es) au niveau d'un bord avant inférieur (22) de ladite coque (2) et une seconde position dans laquelle le(s)dit(es) masque/lunettes de protection (3) est/sont positionné(es) de manière adjacente à une partie arrière (23) de ladite coque (2).

2. Casque de protection (1) selon la revendication 1, **caractérisé en ce que** ledit moyen de raccordement (4) comprend des premiers (42) et des seconds moyens de ressort qui raccordent lesdites première (21) et seconde parties latérales de ladite coque (21) respectivement aux premier (41) et second éléments rigides.

3. Casque de protection (1) selon la revendication 1 ou 2, **caractérisé en ce que** ledit moyen de raccordement (4) comprend un moyen de guidage complémentaire positionné sur lesdites première (21) et seconde parties latérales de ladite coque (2) et sur lesdits premier (41) et second éléments rigides.

4. Casque de protection (1) selon la revendication 3, **caractérisé en ce que** ledit moyen de guidage complémentaire comprend une première (43) et une seconde rainures positionnées respectivement sur lesdits premier (41) et second éléments rigides, et une première (51) et une deuxième broches positionnées respectivement sur lesdites première (21) et seconde parties latérales de ladite coque (2) et insérées de manière coulissante respectivement dans lesdites première (43) et seconde rainures.

5. Casque de protection (1) selon les revendications 2 et 4, **caractérisé en ce que** lesdits premiers moyens de ressort (42) sont raccordés à ladite première broche (51) et audit premier élément rigide (41) et lesdits seconds moyens de ressort sont raccordés à ladite deuxième broche et audit second élément rigide.

6. Casque de protection (1) selon la revendication 5, **caractérisé en ce que** lesdits premier (41) et second éléments rigides comprennent chacun une broche de fixation (61) et un logement (71) pour les premiers (42) et seconds moyens de ressort respectifs.

7. Casque de protection (1) selon la revendication 6, **caractérisé en ce que** lesdites première (51) et deuxième broches sont respectivement positionnées sur une première (50) et une seconde bagues montées en rotation libre respectivement autour d'une troisième (211) et d'une quatrième broches fixées à ladite coque (2), lesdites première (50) et seconde bagues étant insérées et libres de coulisser respectivement dans lesdites première (43) et seconde rainures.

8. Casque de protection (1) selon l'une ou plusieurs des revendications précédentes, **caractérisé en ce que** ledit moyen de raccordement comprend une première (80) et une seconde plaques de guidage comprenant une surface de butée (81) respectivement desdits premier (41) et second éléments rigides.

9. Casque de protection selon la revendication 8, **caractérisé en ce que** ladite surface de butée (81) fait saillie latéralement depuis ladite plaque (80) et est adaptée pour coopérer avec un bord supérieur (413) et inférieur (414) du premier (41) ou second élément rigide correspondant.

10. Casque de protection (1) selon l'une ou plusieurs des revendications précédentes, **caractérisé en ce que** ladite coque (2) comprend un logement (230) dudit/desdites masque/lunettes de protection (3) positionné(es) dans ladite partie arrière (23) de ladite coque (2) au niveau de ladite seconde position dudit/desdites masque/lunettes de protection (3).
